# EUROPEAN PATENT APPLICATION

(11) **EP 1 566 452 A2**
(43) Date of publication of application: **24.08.2005**
(21) Application number: 05003153.3
(22) Date of filing: 15.02.2005
(51) Int. Cl.: C12Q 1/68, G06F 19/00

(54) **Gene information display method and apparatus**

(30) Priority: 17.02.2004 JP 2004040325
(71) Applicant: HITACHI SOFTWARE ENGINEERING CO., LTD., Kanagawa 230-0045 (JP)
(72) Inventor: Matsumoto,Toshiko c/o Hitachi Software Eng. Co Ltd, Tokyo 140-0002 (JP); Nozaki, Yasuyuki c/o Hitachi Software Eng. Co. Ltd, Tokyo 140-0002 (JP); Nakashige, Ryo c/o Hitachi Software Eng. Co. Ltd., Tokyo 140-0002 (JP)
(74) Representative: Liesegang, Roland

(57) **Abstract**

Apparatus and method for appropriately processing gene information in order to allow an easy estimation of haplotypes in large quantities of genome data. A list of genotypes at a plurality of gene loci in a plurality of individuals is displayed, using individual data and gene locus data. The individual data indicates gene loci of individuals and the genotype that exists at each gene locus. The gene locus data indicates a major allele and a minor allele at each gene locus. The individual genotypes are color-coded when they are displayed depending on whether they consist of homozygous major alleles, homozygous minor alleles, or heterozygous major and minor alleles at a particular gene locus.

## Description

### BACKGROUND OF THE INVENTION

### Field of the Invention

The invention relates to a method and apparatus for displaying gene information used in analysis for identifying genes affect phenotypes such as an individual's disease or external characteristics. In particular, the invention relates to a method and apparatus for displaying gene information useful in estimating a haplotype block containing genes as the object of analysis in a genome.

### Description of Related Art

Sequencing of the genomes of humans and animals and plants has progressed and research for analyzing the functions of genes is actively underway. Particular attention is focused on the search for genes affect phenotypes (traits), such as an individual's diseases or external characteristics, in a genome on the basis of linkage disequilibrium analysis, which will be described below.

### Linkage disequilibrium analysis

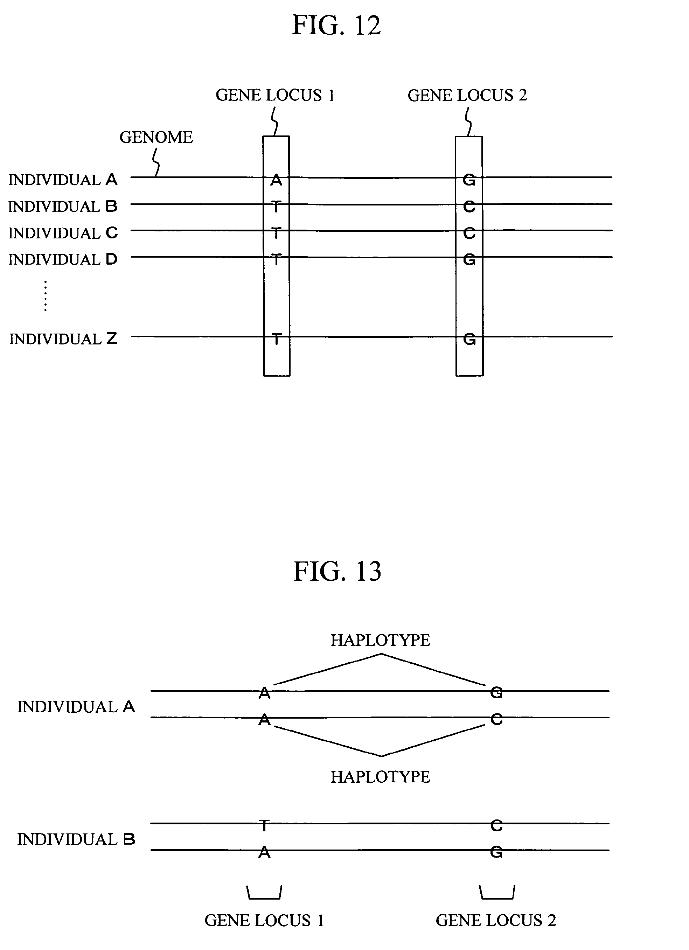

Referring to Fig. 12, a case is considered in which the genomes are compared among individuals A to Z of the same species. Normally, the individuals of the same species possess substantially similar base sequences, with different bases present at several sites. In Fig. 12, the individuals have different bases at gene loci 1 and 2. A gene locus is a specific location on the base sequence of a genome.

Such an occurrence of different forms of a single base on the genome among individuals is called SNP (Single Nucleotide Polymorphism). Normally, one of two kinds of bases (such as A and T) is located at a single gene locus. Very rarely, however, one of three or more types of bases (such as A, T, and G) is located at a single gene locus. In the example shown in Fig. 12, the majority of the individuals have T at gene locus 1, so that T is referred to as major and A is referred to as minor at gene locus 1. Similarly, at gene locus 2, G is major and C is minor.

With reference to Fig. 13, individuals of many living organisms possess a pair of genomes (homologous chromosomes) derived from a female gamete and a male gamete. Genes that exist at the mutually corresponding sites on such a pair of genomes are called alleles, and their combination is called a genotype. As mentioned above, there are portions on the genome where the base sequence is different among individuals, so that any two alleles may be the same in some cases and may be different in other cases. In the example shown in Fig. 13, individual A possesses bases A of the same type at gene locus 1 and possesses different bases G and C at gene locus 2. When attention is focused on genes at a particular site, if there are two alleles of the same kind, they are referred to as homozygous alleles, while if there are two alleles each of a different kind, they are referred to as heterozygous alleles.

When a chromosome is transmitted from a parent to an offspring, a single genome is transmitted by meiosis involving a crossing over, so that a recombination of genes occurs. In general, recombination is more likely to occur in two genes that are spaced apart with a large distance on the genome than genes that are spaced apart with a small distance. When there is a tendency for genes at two gene loci on the genome to be transmitted from a parent to an offspring while the genes are associated with one another, the two gene loci are said to be linked.

On a single genome derived from a male gamete or a female gamete, a combination of alleles that exist at a plurality of gene loci that are linked is called a haplotype. For example, in Fig. 13, when gene locus 1 and gene locus 2 of the genomes of individual A are linked, the individual has haplotype A-G on one of the genomes and haplotype A-C on the other genome. Thus, an individual that has a set of two homologous genomes always has a set of two haplotypes, and such a set (pair) of haplotypes is called a diplotype.

In a plurality of linked gene loci, a phenomenon is sometimes observed where the frequency of a specific haplotype is vastly different from the frequency obtained by multiplying the frequencies of alleles at each gene locus (namely, the distribution of alleles is not independent between a plurality of gene loci). In this case, the gene loci are said to be in linkage disequilibrium.

As described above, analysis of linkage disequilibrium enables the search for genes affect phenotypes (traits), such as an individual's disease or external characteristics, in a genome. In a single population, it is estimated that many of the disease-causing genes responsible for a disease with a relatively high frequency are derived from mutation in a common ancestral gene (the "common disease common variant hypothesis"). Then, it can be expected that an SNP allele near the gene locus at which the mutation had occurred has also been transmitted together with the disease-causing gene. Namely, it can be expected that there is linkage disequilibrium between the gene locus of the disease-causing gene and the peripheral SNP gene locus. Such a region on the genome is referred to as a linkage disequilibrium block or a haplotype block. A search for a haplotype block commonly possessed by individuals with a specific disease enables the identification of genes responsible for the disease.

### Estimation of a haplotype

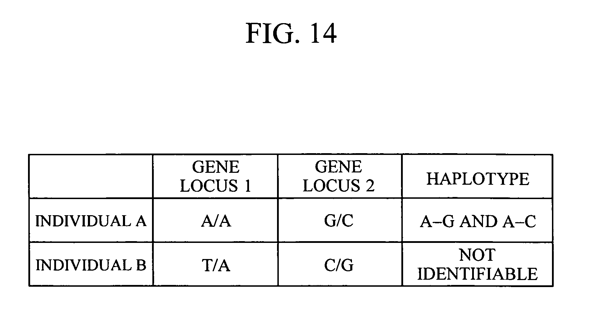

When analyzing genes, generally a pair of alleles, namely a genotype, at each gene locus is identified. Results from such analysis, however, do not always shed light on a haplotype. For example, with reference to Fig. 13, once it is determined that individual A has a genotype A/A at gene locus 1 and a genotype G/C at gene locus 2, it can be learned that the haplotype on one genome is A-G and the haplotype on the other genome is A-C. However, even if it is determined that individual B has a genotype T/A at gene locus 1 and a genotype C/G at gene locus 2, this analysis result does not allow the haplotypes possessed by individual B to be uniquely identified (see Fig. 14).

In general, even if all of the genotypes of an individual were to be identified, it would not always lead to the identification of the haplotypes possessed by that individual. In such a case, one says "the phase cannot be identified." Although it is possible to carry out an experiment to directly identify the haplotype, such an experiment would have to be very sophisticated and complicated, requiring a great amount of time and cost. Therefore, when performing a linkage disequilibrium analysis on the basis of the deviation of haplotype frequency, a technique is employed whereby the haplotype is estimated using software. Examples of the algorithm for estimating haplotypes include the EM algorithm and the Clark algorithm.

Non-Patent Documents 1 and 2 disclose methods of identifying haplotypes, showing identification results in the drawings. Non-Patent Document 3 discloses that statistical quantities are determined or a linkage disequilibrium map is created based on haplotype estimation results. An example of the software for performing these processes on a computer is ARLEQUIN.
Non-Patent Document 1: Daly, M. J. et. al., Nature Genetics Volume 29, October 2001 (particularly Fig. 2)
Non-Patent Document 2: Patil, N. et al., Science vol. 294, 23 November, 2001 (particularly Fig. 2)
Non-Patent Document 3: Taillon-Miller, P. et al., Nature Genetics Volume 25, July 2000 (particularly Fig. 2)

### SUMMARY OF THE INVENTION

The conventional haplotype estimation techniques using software require a large storage capacity and much time in program execution, and they can take too much time or the software can even become inoperative when the experimental data required to be processed is large. The problem is particularly serious when the EM algorithm is used for the haplotype estimation because in this case the required storage capacity increases exponentially as the number of the gene loci that are the objects of estimation increases.

Further, when it is desired to repeatedly carry out an analysis of linkage disequilibrium while changing the data set, such as the gene loci or individuals, as the analysis objects, the conventional techniques require the aforementioned haplotype estimation to be carried out each time a new data set is entered, thus requiring more processing time.

It is therefore an object of the invention to provide a method and apparatus for processing and displaying gene information in an appropriate manner so that haplotype estimation can be performed easily on the basis of a large volume of genome data.

The inventors' research and analysis has led to the discovery that, focusing on the genotype at a gene locus where an SNP appears, a haplotype block can be estimated by comparing individuals as analysis objects on the basis of what kind of combination of major and minor alleles the genotype consists of at the gene locus.

In one aspect, the invention provides a method of displaying a list of genotypes at a plurality of gene loci in a plurality of individuals on the basis of individual data and gene locus data, using a gene information display apparatus comprising a display unit for displaying said list of genotypes and a memory unit in which said individual data and said gene locus data are stored, said individual data indicating the gene loci of each individual and the genotypes that exist at said gene loci, and said gene loci data indicating a major allele and a minor allele at each gene locus, wherein said genotypes are color-coded when they are displayed depending on whether they consist of homozygous major alleles, homozygous minor alleles, or heterozygous major and minor alleles at each gene locus.

In the gene display method of the invention, a genotype consisting of heterozygous major and minor alleles is assigned an intermediate color between a color assigned to the genotype consisting of homozygous major alleles and a color assigned to the genotype consisting of homozygous minor alleles.

Since the three kinds of genotypes are color-coded in a stepwise manner when they are displayed in accordance with the frequency of appearance of the alleles, the user can intuitively recognize the state of existence of the genotype at each gene locus.

In the gene display method of the invention, from each said individual data, only data that pertains to one or more specific gene loci is selectively displayed.

Preferably, the individuals are rearranged after selectively displaying only those gene loci that can be considered to be a haplotype block because individuals possessing an identical genotype differ from one haplotype block to another.

In the gene display method of the invention, only that data in said individual data that pertains to one or more specific individuals is selectively displayed.

It is thought that the reliability of haplotype block estimation can be improved by extracting only those individuals that have common population attributes in cases where the individual data include different population attributes.

In the gene display method of the invention, the individuals are rearranged on the basis of the kind of genotype at one or more specific gene loci when they are displayed.

In this way, those gene loci with similar states of existence of genotypes to that at one or more specific gene loci as a reference of rearrangement can be visually recognized when they are displayed, thus facilitating haplotype estimation.

In another aspect, the invention provides a gene information display apparatus comprising:
a display unit;
a memory unit in which individual data and gene locus data are stored, said individual data indicating gene loci of each individual and the genotype that exists at each gene locus, and said gene locus data indicating a major allele and a minor allele at each gene locus;
a data edit processing unit for editing the individual data and gene locus data stored in said memory unit; and
a data display unit for causing said display unit to display a list of the genotypes at a plurality of gene loci in a plurality of individuals on the basis of the individual data and gene locus data stored in said memory unit, wherein:
   said data display unit displays the individual genotypes that are color-coded depending on whether they consist of homozygous major alleles, homozygous minor alleles, or heterozygous major and minor alleles at each gene locus.

In the gene information display apparatus of the invention, said data display unit displays the genotype consisting of heterozygous major and minor alleles using an intermediate color between a color assigned to the genotype of homozygous major alleles and a color assigned to the genotype of homozygous minor alleles.

In the gene information display apparatus of the invention, said data display unit selectively displays only that data that pertains to one or more specific gene loci from said individual data.

In the gene information display apparatus of the invention, said data display unit selectively displays only that data in said individual data that pertains to one or more specific individuals.

In the gene information display apparatus of the invention, said data edit processing unit rearranges the individuals on the basis of the kind of the genotype at one or more specific gene loci.

In the gene information display apparatus of the invention, said data edit processing unit defines a plurality of gene loci as a single block in accordance with user operation, and said memory unit stores such a defined block as gene locus data.

In accordance with the inventive method and apparatus for displaying gene information, haplotype blocks that can be considered to be in linkage disequilibrium can be estimated without requiring a highly sophisticated technique for determining or estimating haplotype blocks from the genome data of each individual. The method and apparatus of the invention further allow haplotype blocks to be estimated much more easily and faster than the conventional haplotype block estimation techniques even when large quantities of genome data must be processed. In particular, in accordance with the invention, the gene loci or individuals as the object of analysis can be freely changed or rearranged without requiring a repetition of a complicated computation. Accordingly, the user can perform haplotype estimation by using various conditions through a trial and error.

### BRIEF DESCRIPTION OF THE DRAWINGS

Fig. 1 shows a functional block diagram schematically illustrating the system configuration of the gene information display apparatus of the invention.
Fig. 2 shows an example of the data structure of individual data stored in a data memory in the gene information display apparatus of the invention.
Fig. 3 shows an example of the data structure of gene locus data stored in the data memory in the gene information display apparatus of the invention.
Fig. 4 schematically shows gene information displayed on the screen of a display unit of the gene information display apparatus of the invention, showing a list of genotype data regarding the entire individuals and gene loci contained in the data memory of the gene information display apparatus.
Fig. 5 schematically shows gene information displayed on the screen of the display unit of the gene information display apparatus, showing a list of genotype data regarding only those gene loci selected by the user.
Fig. 6 schematically shows gene information displayed on the screen of the display unit of the gene information display apparatus, showing the result of rearranging the individuals on the screen of Fig. 5 in accordance with a specific gene locus.
Fig. 7 shows a schematic flowchart of the process of displaying and editing gene information in the gene information display apparatus of the invention.
Fig. 8 shows a detailed flowchart of the process of rearranging the individuals based on a genotype at one or more specific gene loci in the gene information display apparatus of the invention.
Fig. 9 shows a display of a list of gene information similar to the one shown in Fig. 4, using the experimental data published in a paper according to Non-Patent Document 1 (Daly, M. J., et al., 2001).
Fig. 10 shows a display of gene information regarding gene loci 1 to 18 that have been extracted from the gene information displayed in Fig. 9.
Fig. 11 shows a display of the result of rearranging the individuals in the gene information displayed in Fig. 10 in accordance with the genotype at gene locus 2.
Fig. 12 is a drawing for explaining SNP that appears on a genome.
Fig. 13 is a drawing for explaining a haplotype of SNP that appears on homologous genomes.
Fig. 14 shows a drawing for explaining a method of identifying a haplotype based on information about a genotype at a plurality of gene loci.

### DETAILED DESCRIPTION OF THE PREFERRED EMBODIMENTS

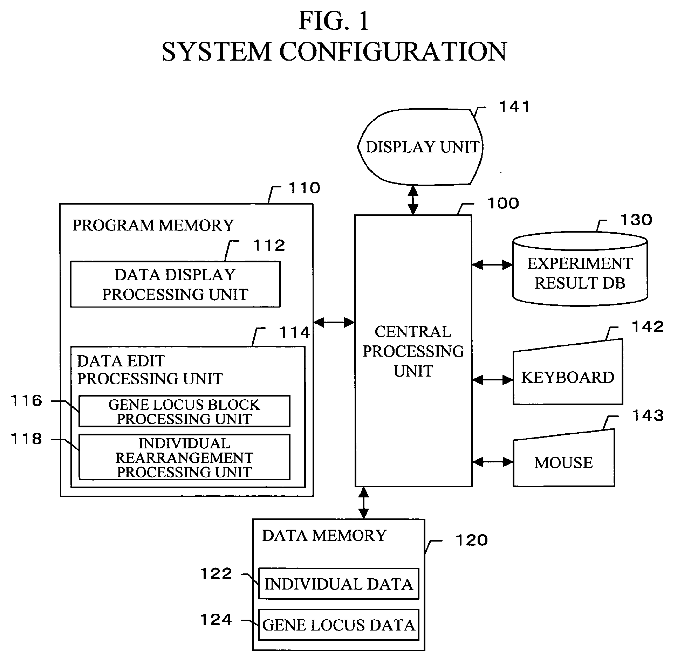

The method and apparatus for displaying gene information in accordance with the invention will be hereafter described in detail by way of a preferred embodiment thereof, with reference made to the drawings, of which Figs. 1 to 11 show the embodiment of the invention. Like reference numerals identify identical elements with basically the same structure and function throughout the several views.

### Structure of the gene information display apparatus

Fig. 1 is a functional block diagram schematically illustrating the system configuration of the gene information display apparatus according to the invention. The gene information display apparatus of the invention comprises a central processing unit 100, a program memory 110, a data memory 120, an experiment result database 130, a display unit 141, and an input/output device including a keyboard 142 and a mouse 143.

The central processing unit 100 is a processing unit made up of a CPU or a MPU, by which the operation of each element of the apparatus is controlled, necessary calculations are performed, and data communications between the individual elements are controlled. The program memory 110 is a memory for storing a program for processes in the central processing unit 100, and fixed data, such as control data, for example. Typically, the program memory 110 is a read-only memory (ROM) made up of semiconductor memory elements. The data memory 120 is a memory for the temporary storage of data utilized by the central processing unit 100, for example. Typically, the data memory 120 is a random-access memory (RAM) made up of semiconductor memory elements or magnetic memory elements. The central processing unit 100 utilizes the data memory 120 as a working memory in accordance with the operating program stored in the program memory 110 so as to carry out processes necessary for control, calculations, and data transmission/reception.

The experiment result database 130 stores experimentally obtained gene information regarding a plurality of individuals. Specifically, the information concerns the gene loci where an SNP appears and the genotypes that exist at those gene loci. The experiment result database 130 also stores the population attributes (race, disease, physical characteristics, and so on) of each individual, and stores major and minor alleles at each gene locus in each population. In actual experimental data, some genotype data may in some cases be lacking, due to experimental failure or the like. Such lacking data is stored as missing.

The display unit 141 is an output device for displaying gene information to the user, and it may include a CRT or an LCD. The keyboard 142 and mouse 143 are input devices through which the user can enter operational inputs to the gene information display apparatus.

In the gene information display apparatus according to the invention, the data memory 120 stores individual data 122 including the genome information regarding each individual. The data memory 120 also stores gene locus data 124 including information about the frequency of alleles on each gene locus on the genome and information about blocking (formation of a block). The individual data 122 and the gene locus data 124 can be acquired from the experiment result database 130 as needed.

In the gene information display apparatus of the invention, the program memory 110 includes a data display processing unit 112 for carrying out processes for displaying gene data on the display unit 141, and a data edit processing unit 114 for editing the object of display in accordance with the operational inputs from the user. The data edit processing unit 114 further includes a gene locus blocking process unit 116 for bundling a plurality of gene loci together as a block, and an individual rearrangement processing unit 118.

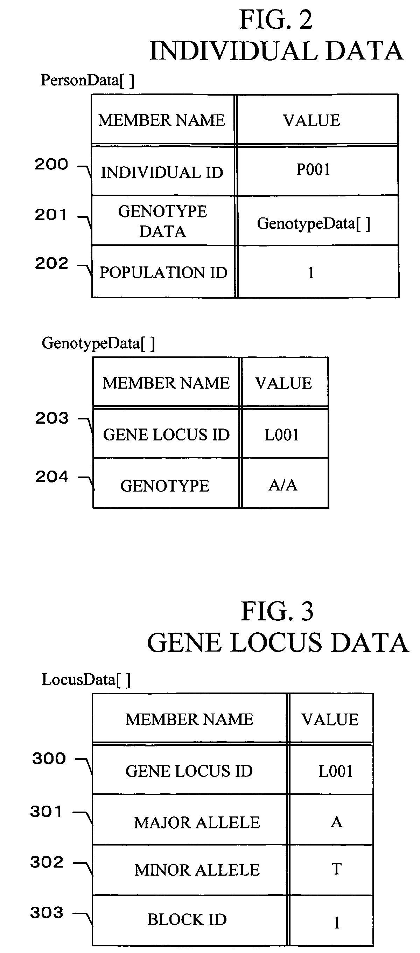

Fig. 2 shows an example of the data structure of the individual data 122 stored in the data memory 120. The individual data 122 carries the genome data of each individual in the form of a PersonData structure and a GenotypeData structure. The PersonData structure includes an ID 200 for identifying the individual, data 201 regarding the genotype of each individual, and an ID 202 of the population to which each individual belongs. The genotype data 201 is further defined in terms of a GenotypeData structure, which includes an ID 203 for identifying the gene locus and data regarding the genotype at each gene locus. Each record in the GenotypeData structure corresponds to a single gene locus. The gene locus ID 203 represents the physical positional relationship on the genome. For example, gene loci L001, L002, L003, ... are also arranged in this order on the genome.

Fig. 3 shows an example of the data structure of the gene locus data 124 stored in the data memory 120. The gene locus data 124 is carried in the form of a LocusData structure, which includes an ID 300 for identifying the gene locus, data 301 regarding a major allele and data 302 regarding a minor allele on each gene locus, and a block ID 303 indicating the block that is allocated to each gene locus.

### Method of displaying gene information

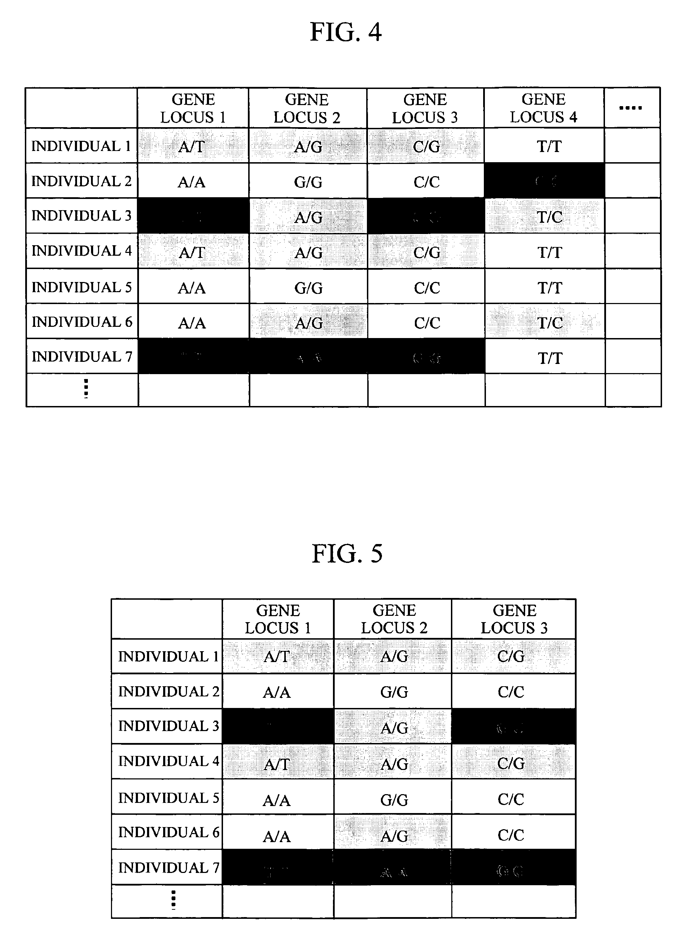

A method of displaying gene information in the gene information display apparatus of the invention that is configured as described above will be described. Figs. 4 to 6 schematically show gene information that is displayed on the screen of the display unit 141 of the gene information display apparatus shown in Fig. 1. In these screens, the individuals are shown along the vertical axis and the gene loci are shown along the horizontal axis, and the genotypes of specific individuals at specific gene loci are listed in a table. It should be noted, however, that the manner of display is obviously not limited to that of the herein-illustrated example.

On the screen shown in Fig. 4, there is displayed a list of the genotype data regarding the entire individuals and gene loci that are included in the data read into the data memory 120 of the gene information display apparatus. The rows and columns in the table are arranged in ascending order of the individual ID and gene locus ID, respectively. Each cell in the table is color-coded in accordance with the genotype data. Specifically, the color coding is based on whether the genotype consists of homozygous major alleles, homozygous minor alleles, or heterozygous major and minor alleles at a particular gene locus. In particular, homozygous major alleles and homozygous minor alleles are allocated with different colors (gray and white in the drawings), and an intermediate color (light gray in the drawings) is allocated to heterozygous major and minor alleles. Thus, different colors may be allocated to the same genotype at different gene loci.

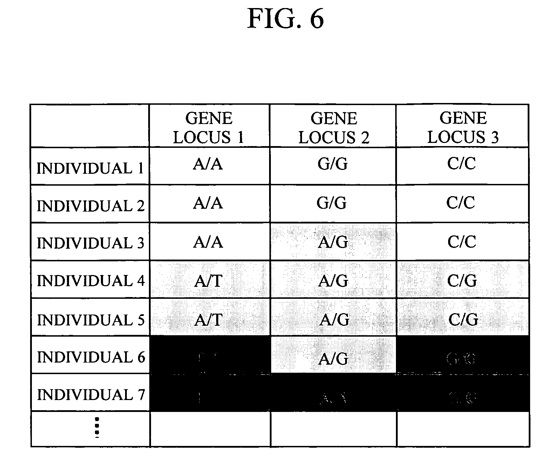

On the screen shown in Fig. 5, the genotype data that is displayed in a list only pertains to those gene loci (loci 1, 2, and 3 in the illustrated example) that have been selected by the user from the table of Fig. 4. The user can further rearrange the individuals on the screen of Fig. 5 on the basis of the genotype at one or more specific gene loci. For example, Fig. 6 shows the result of rearranging the individuals on the basis of the genotype at gene locus 1 among the gene loci displayed on the screen. The rearrangement is carried out such that the individuals are arranged either in order of those with homozygous major alleles, those with heterozygous major and minor alleles, and those with homozygous minor alleles at gene locus 1, or in the opposite order.

In accordance with the display of Fig. 6, it is seen that:
those individuals with homozygous major alleles at gene locus 1 also have homozygous major alleles at gene locus 3;
those individuals with heterozygous major and minor alleles at gene locus 1 also have heterozygous major and minor alleles at gene locus 3; and
those individuals with homozygous minor alleles at gene locus 1 also have homozygous minor alleles at gene locus 3.

It is also seen that with regard to gene locus 2 too, the individuals have substantially identical genotypes to those at gene locus 1. Accordingly, it can be estimated that gene loci 1, 2, and 3 are in a state of strong linkage disequilibrium. Namely, it can be predicted that gene loci 1,2, and 3 make up a single haplotype block. While the estimation of haplotype blocks is normally visually done by the user, the gene information display apparatus of the invention may be provided with a function whereby a region that is estimated to be a haplotype block is automatically recognized and presented to the user.

The user, using the keyboard 142 and mouse 143 of the gene information display apparatus, can then designate gene loci 1, 2, and 3 on the display screen and cause them to be stored as a single haplotype block. By this operation, the same block ID is written into the LocusData for gene loci 1, 2, and 3 included in the gene locus data 124 in the data memory 120.

While in the above-described example the entire individuals included in the individual data 122 on the data memory 120 are displayed in a list, it is also possible to selectively display some of the individuals.

### Flow of the process for displaying gene information

Hereafter the software process of displaying the aforementioned gene information in the gene information display apparatus of the invention will be described.

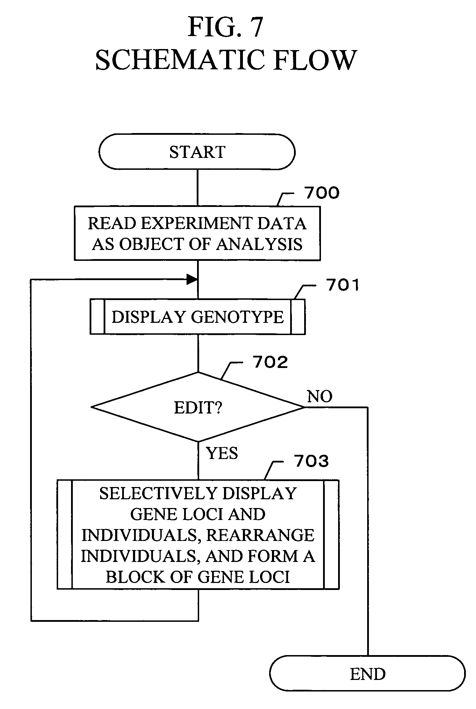

Fig. 7 shows a flowchart illustrating the outline of the processes for displaying and editing gene information. The gene information display apparatus first reads experiment-result data as the object of analysis from the experiment result database 130 (step 700). The data thus read is stored in the data memory 120 in terms of the individual data 122 and the gene locus data 124. Then, the gene information display apparatus displays on the display unit 141 a list of the genotype at each gene locus in each individual (as shown in Fig. 4) based on the data stored in the data memory 120 (step 701). The cells are color-coded depending on whether the genotype consists of homozygous major alleles, homozygous minor alleles, or heterozygous major and minor alleles, or whether the relevant data is missing. Which alleles are major or minor at each gene locus can be determined on the basis of the information in the experiment result database 130 if available therein, or, if not available, based on the data in the data memory 120.

The user can then edit the data displayed on the screen (step 702). Specifically, on the screen shown in Fig. 4, the user can selectively display specific gene loci or specific individuals, or rearrange the individuals based on the genotype at one or more specific gene loci. The user can further designate a plurality of gene loci and store them as a single haplotype block ("blocking") (step 703). After these processes have been performed, the screen after editing is displayed by the process in step 701 (whereby the screen shown in Fig. 5 or 6 is displayed).

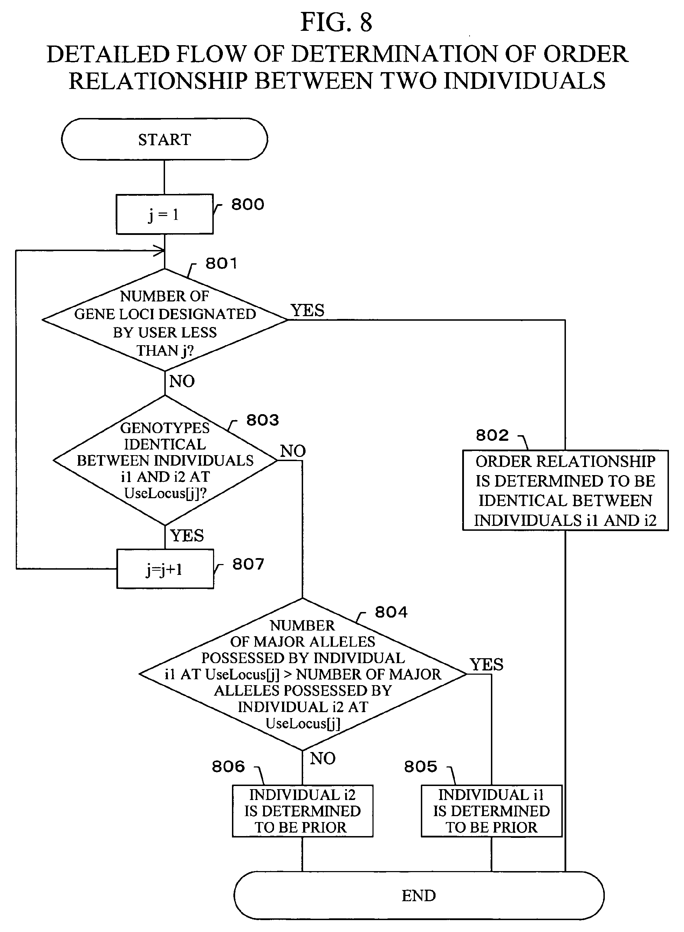

Fig. 8 shows a flowchart illustrating in detail the process of rearranging the individuals based on the genotype at a specific gene locus in step 703 of Fig. 7. In the illustrated example, the order relationship between two individuals is determined on the basis of one or more gene loci designated by the user. The individual data regarding the two individuals consist of PersonData [i1] and PersonData [i2], and the gene loci designated by the user are presumed to be stored in a sequence UseLocus [].

First, a variant j indicating the element number of the array UseLocus [] is initialized to 1 (step 800). Then, it is examined whether the number of gene loci designated by the user is less than j (step 801). If the number of gene loci designated by the user is less than j, this means however all of the gene loci designated by the user have been referenced, the order relationship between individuals i1 and i2 has not been determined. Thus, in such a case, it is determined that the order relationship is equal, and the process comes to an end (step 802).

If it is determined in step 801 that the number of gene loci designated is j or more, the genotypes of individuals i1 and i2 in the gene locus UseLocus [j] are compared (step 803). If the two genotypes are not identical, the number of major alleles possessed by individuals i1 and i2 in the gene locus UseLocus [j] is compared (step 804). For example, if, at the gene locus UseLocus [j], individual i1 has homozygous major alleles and individual i2 has heterozygous major and minor alleles, this shows that individual i1 has more major alleles than individual i2. Then, the individual that has been determined to possess a greater number of major alleles is determined to be preceding in the order relationship, and the process comes to an end (steps 805 and 806).

If in step 803 the genotypes of the two individuals at the gene locus [j] are identical, j is incremented and the process is repeated from step 801 (step 807). By carrying out the above-described processes between the individuals as the object of display, the individuals can be rearranged in accordance with the genotype at the gene loci designated by the user.

### Example

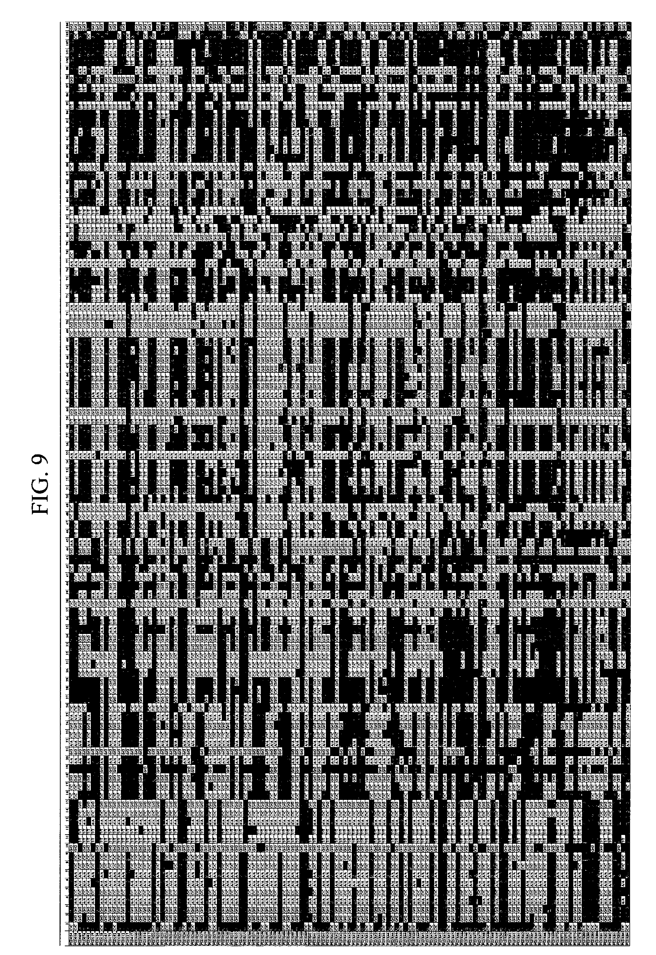

Fig. 9 shows a displayed list of gene information similar to the one shown in Fig. 4, using the experimental data published in Non-Patent Document 1 (Daly, M. J., et al., 2001) (corresponding to the data stored in the experiment result database 130 in the gene information display apparatus of the invention). In this list, homozygous major alleles are shown in white, homozygous minor alleles are shown in red, heterozygous major and minor alleles are shown in an intermediate color between red and white, namely pink, and the missing data is shown in gray.

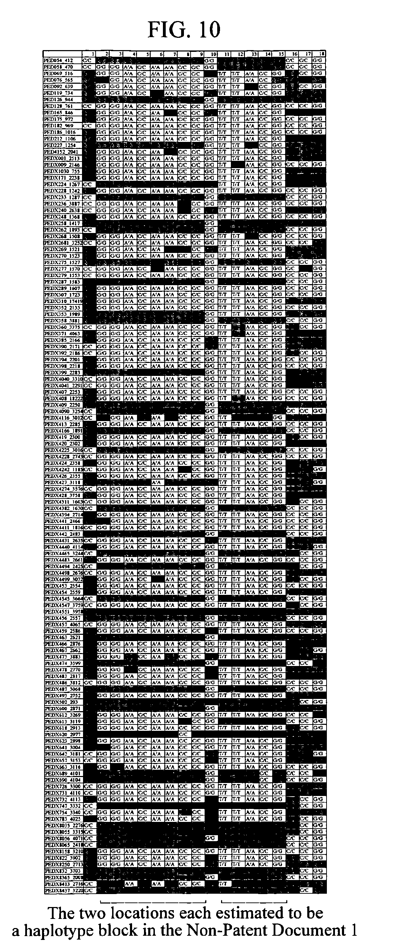

Fig. 10 shows the gene information for gene loci 1 to 18 selected from the gene information shown in Fig. 9. Since the individuals having the same genotype differ from one haplotype block to another, the individuals are preferably rearranged after those gene loci that can be considered to be a haplotype block have been selectively displayed. Fig. 10 apparently indicates that many individuals share common genotypes at gene loci 2 to 9 and gene loci 11 to 15. In order to corroborate this initial determination, the individuals were then rearranged on the basis of the genotype at gene locus 2.

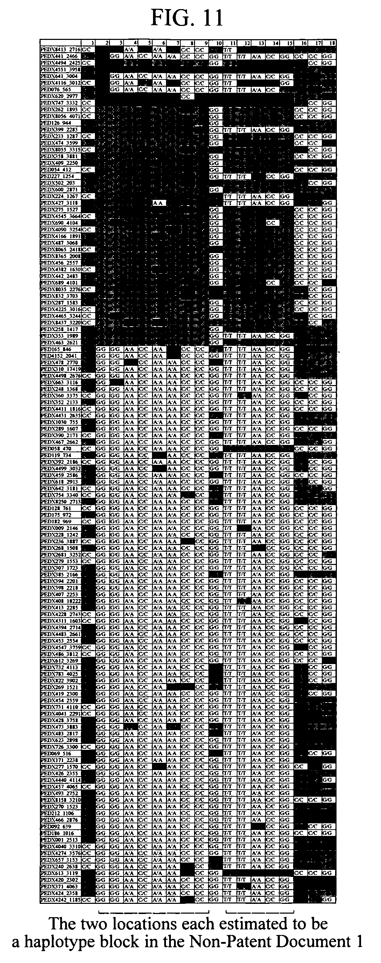

Fig. 11 shows the display of the result of rearranging the individuals with regard to the gene information shown in Fig. 10 on the basis of the genotype at gene locus 2. The result clearly shows that the individuals possess substantially identical genotypes at gene loci 2 to 9 and at gene loci 11 to 15. The same two locations are also estimated to be haplotype blocks by a conventional haplotype estimation method in the paper of Non-Patent Document 1.

While in the above-described embodiment an SNP has been utilized in which two kinds of alleles exist at a single gene locus, the method of the invention can also be applied to an SNP in which three or more kinds of alleles exist at one gene locus. For example, when three kinds of alleles exist at a single gene locus, red, blue, and yellow may be assigned to the three kinds of homozygous alleles, respectively, purple to first and second heterozygous alleles, green to second and third heterozygous alleles, and orange to first and third heterozygous alleles when they are displayed.

It is also conceivable to appropriately color-code genotypes of species in which chromosomes form triploids or tetraploids in accordance with the method of the invention.

While a preferred embodiment of the invention has been described using specific terms, such description is for illustrative purposes only, and it is to be understood that changes and variations may be made without departing from the spirit or scope of the invention.

The method and apparatus for displaying gene information in accordance with the invention can be utilized for estimating haplotype blocks on the genome of humans, other animals and plants, and other species in which chromosomes form diploids, tripoids, or tetrapoids, for example.

## Claims

1. A method of displaying a list of genotypes at a plurality of gene loci in a plurality of individuals on the basis of individual data and gene locus data, using a gene information display apparatus comprising a display unit for displaying said list of genotypes and a memory unit in which said individual data and said gene locus data are stored, said individual data indicating the gene loci of each individual and the genotypes that exist at said gene loci, and said gene loci data indicating a major allele and a minor allele at each gene locus, wherein said genotypes are color-coded when they are displayed depending on whether they consist of homozygous major alleles, homozygous minor alleles, or heterozygous major and minor alleles at each gene locus.

2. The method according to claim 1, wherein a genotype consisting of heterozygous major and minor alleles is assigned an intermediate color between a color assigned to the genotype consisting of homozygous major alleles and a color assigned to the genotype consisting of homozygous minor alleles.

3. The method according to claim 1 or 2, wherein from each said individual data, only data that pertains to one or more specific gene loci is selectively displayed.

4. The method according to claim 1 or 2, wherein only that data in said individual data that pertains to one or more specific individuals is selectively displayed.

5. The method according to any one of claims 1 to 4, wherein the individuals are rearranged on the basis of the kind of genotype at one or more specific gene loci when they are displayed.

6. A gene information display apparatus comprising:
a display unit;
a memory unit in which individual data and gene locus data are stored, said individual data indicating gene loci of each individual and the genotype that exists at each gene locus, and said gene locus data indicating a major allele and a minor allele at each gene locus;
a data edit processing unit for editing the individual data and gene locus data stored in said memory unit; and
a data display unit for causing said display unit to display a list of the genotypes at a plurality of gene loci in a plurality of individuals on the basis of the individual data and gene locus data stored in said memory unit, wherein:
said data display unit displays the individual genotypes that are color-coded depending on whether they consist of homozygous major alleles, homozygous minor alleles, or heterozygous major and minor alleles at each gene locus.

7. The gene information display apparatus according to claim 6, wherein said data display unit displays the genotype consisting of heterozygous major and minor alleles using an intermediate color between a color assigned to the genotype of homozygous major alleles and a color assigned to the genotype of homozygous minor alleles.

8. The gene information display apparatus according to claim 6 or 7, wherein said data display unit selectively displays only data that pertains to one or more specific gene loci from each said individual data.

9. The gene information display apparatus according to claim 6 or 7, wherein said data display unit selectively displays only that data in said individual data that pertains to one or more specific individuals.

10. The gene information display apparatus according to any one of claims 6 to 9, wherein said data edit processing unit rearranges the individuals on the basis of the kind of the genotype at one or more specific gene loci.

11. The gene information display apparatus according to any one of claims 6 to 10, wherein said data edit processing unit defines a plurality of gene loci as a single block in accordance with user operation, and said memory unit stores such a defined block as gene locus data.
